# EUROPÄISCHE PATENTANMELDUNG

(11) **EP 2 407 182 A1**
(43) Veröffentlichungstag der Anmeldung: **18.01.2012**
(21) Anmeldenummer: 11173909.0
(22) Anmeldetag: 14.07.2011
(51) Int. Cl.: A61L 2/20, B65B 55/02, B67C 7/00

(54) **Vorrichtung zum Sterilisieren von Behältnissen**

(30) Priorität: 16.07.2010 DE 102010027494
(71) Anmelder: Krones AG, 93073 Neutraubling (DE)
(72) Erfinder: Söllner, Jürgen, 93176 Beratzhausen (DE); Dahmen, Michael, 22527 Hamburg (DE)
(74) Vertreter: Hannke, Christian

(57) **Zusammenfassung**

Eine Vorrichtung (1) zum Sterilisieren von Verpackungsmitteln und insbesondere Behältnissen mit einer Verdampfereinheit (2), welche eine Zuführungseinrichtung (4) für ein Sterilisationsmittel und eine Zuführleitung (6) für ein gasförmiges Medium aufweist, wobei die Verdampfereinheit das Sterilisationsmittel in das gasförmige Medium einbringt und weiterhin eine Abführeinrichtung (82) zum Abführen einer Sterilisationsmischung aus dem Sterilisationsmittel und dem gasförmigen Medium aufweist und mit einer ersten Beaufschlagungseinrichtung (12), welche wenigstens einen ersten Bereich mit der Sterilisationsmischung beaufschlagt, wobei die erste Beaufschlagungseinrichtung mit der Verdampfereinheit (2) in Strömungsverbindung steht. Erfindungsgemäß weist die Vorrichtung (1) eine zweite Beaufschlagungseinheit (14) aufweist, welche wenigstens einen zweiten sich von dem ersten Bereich unterscheidenden Bereich mit der Sterilisationsmischung beaufschlagt, wobei auch die zweite Beaufschlagungseinrichtung mit der Verdampfereinheit (2) in Strömungsverbindung steht.

## Beschreibung

Die vorliegende Erfindung bezieht sich auf eine Vorrichtung zum Sterilisieren von Verpackungsmitteln und insbesondere von Behältnissen. In der getränkeherstellenden Industrie ist es seit langem bekannt, für bestimmte Zwecke die zu befüllenden Behältnisse vor deren Befüllung zu sterilisieren. Eine Möglichkeit dieser Sterilisation besteht darin, dass die Behältnisse mit einem Sterilisationsmittel, wie insbesondere H₂O₂, ggfs. aber auch Peressigsäure beaufschlagt und auf diese Weise sterilisiert werden. Insbesondere bei kaltaseptischen Abfüllanlagen wird im Regelfall ein steriles Produkt in vorher sterilisierte Behältnisse abgefüllt und dann diese mit einem sterilisierten Verschluss verschlossen. Die Sterilisation der Verpackungsmaterialien und die Abfüllung des Produkts finden dabei meist in einer Maschineneinheit statt. Der gesamte Raum, in dem die Sterilisation und auch die Abfüllung und das Verschließen stattfinden, wird oft als Isolator bezeichnet.

Der Packstoff bzw. die Behältnisse selbst werden im Produktionszyklus kontinuierlich sterilisiert. Zudem ist es jedoch auch oftmals erforderlich, dass der genannte Isolator zu Beginn der Produktion selbst sterilisiert wird. Im Stand der Technik wurde diese Oberflächenentkeimung im Isolator üblicherweise durch flüssige Agenzien vorgenommen, bevorzugt wurde hierzu eine Peressigsäurelösung eingesetzt. Diese Lösung wurde in definierter Konzentration aufgetragen und für eine bestimmte Zeit (auch als Kontaktzeit bezeichnet) einwirken gelassen. Anschließend wird die Lösung mit sterilem Wasser abgespült.

Im internen Stand der Technik der Anmelderin ist man darüber hinaus dazu übergegangen, die Oberfläche des Isolators mit einem Gasgemisch aus Luft und verdampftem Wasserstoffperoxid (H₂O₂) zu entkeimen. Neben H₂O₂ kann jedoch auch beispielsweise Peressigsäure verdampft und als Sterilisationsmittel eingesetzt werden. Daneben wäre auch eine Behandlung mit Ozon und/oder anderen flüssigen oder gasförmigen Sterilisationsmitteln denkbar.

Das flüssige Sterilisationsmedium wird dabei bei einer bekannten Vorgehensweise auf eine heiße Oberfläche aufgesprüht und dabei verdampft. Vorbeiströmender Luftvolumenstrom nimmt dieses Gas mit. Diese Mischung wird dem Isolator oder aber einer Behandlungseinheit zugeführt und sterilisiert anschließend den Isolator oder auch Packstoffe bzw. Behältnisse.

Zu diesem Zweck wird für jede Art der Behandlung, d. h. die Behandlung der Behältnisse selbst, der Verschlüsse, oder auch des Isolators je ein individueller Verdampfer eingesetzt. Diese Verdampfer weichen in ihrer Größe stark ab, da z. B. für die Isolatorsterilisation in etwa die zehnfache Menge an Gas benötigt wird, wie für die Behältnissterilisation.

Aus der DE 38 19 419 A1 ist eine Zumesseinrichtung für Dosiervorrichtungen, insbesondere zur Sterilisation von Verpackungsmaterial bekannt. Dabei ist ein Dosiergehäuse mit einem verstellbar angeordneten Steuerschieber vorgesehen, um auf diese Weise eine Dosierung des Sterilisationsmittels erreichen zu können.

Die DE 10 2007 039 010 A1 beschreibt ein Dosier- und Versorgungssystem der Vorrichtung zum H₂O₂-Sterilisieren von Packmitteln sowie eine Vorrichtung mit einem solchen Versorgungssystem. Dabei ist neben einem das Wasserstoffperoxid bereitstellenden Vorratsbehälter eine steuerbare Verbindung zwischen dem Vorratsbehälter und einem Behandlungskopf vorgesehen, wobei die steuerbare Verbindung von wenigstens einer Ringleitung gebildet ist, die zusammen mit dem Vorratsbehälter einen Kreis oder Umlauf für das Wasserstoffperoxid bildet, wobei der Behandlungskopf über wenigstens ein Dosierventil an die Ringleitung angeschlossen ist.

Aus der DE 689 01 685 T2 ist ein Verfahren zum Sterilisieren einer Verpackungsmaschine mit einem flüssigen Desinfektionsmittel bekannt. Dabei wird ein Desinfektionsmittel in einen überhitzten Luftstrom gespritzt und während eines vorbestimmten Zeitintervalls dadurch ein Verdampfen des flüssigen Desinfektionsmittels erreicht. Anschließend wird das Gemisch aus überhitzter Luft und verdampftem flüssigen Desinfektionsmittel durch die wärmespeichernde Rohrleitung und in Kontakt mit den zu sterilisierenden Bauteilen gebracht.

Der vorliegenden Erfindung liegt die Aufgabe zugrunde, insbesondere die Herstellungskosten für derartige Vorrichtungen zum Sterilisieren von Behältnissen zu reduzieren.

Diese Aufgabe wird durch die Gegenstände der unabhängigen Ansprüche erreicht. Vorteilhafte Ausführungsformen und Weiterbildungen sind Gegenstand der Unteransprüche.

Eine erfindungsgemäße Vorrichtung zum Sterilisieren von Verpackungsmitteln und insbesondere von Behältnissen weist eine Verdampfereinheit auf, welche eine Zuführungseinrichtung für ein Sterilisationsmittel und eine Zuführleitung für ein gasförmiges Medium aufweist. Dabei bringt die Verdampfereinheit das Sterilisationsmittel in das gasförmige Medium ein und es ist weiterhin eine Abführeinrichtung vorgesehen, um eine Sterilisationsmischung aus dem Sterilisationsmittel mit dem gasförmigen Medium abzuführen.

Weiterhin ist eine erste Beaufschlagungseinrichtung vorgesehen, welche wenigstens einen ersten vorgegebenen Bereich (insbesondere des Verpackungsmittels und/oder von Elementen der Vorrichtung wie beispielsweise von Greifelementen zum Halten der Behältnisse) mit der Mischung beaufschlagt, wobei die erste Beaufschlagungseinrichtung mit der Verdampfereinheit in Strömungsverbindung bezüglich der Sterilisationsmischung steht.

Erfindungsgemäß weist die Vorrichtung eine zweite Beaufschlagungseinheit auf, welche wenigstens einen zweiten vorgegebenen Bereich (insbesondere des Verpackungsmittels und/oder von Elementen der Vorrichtung wie beispielsweise von Greifelementen zum Halten der Behältnisse), der sich von dem ersten Bereich unterscheidet mit der Mischung beaufschlagt, wobei auch die zweite Beaufschlagungseinrichtung mit der Verdampfeinheit in Strömungsverbindung steht.

Es wird daher erfindungsgemäß vorgeschlagen, lediglich einen Verdampfer vorzusehen, mittels dessen beide genannten Bereiche sterilisiert werden. Bei der Verdampfereinheit kann es sich um eine beliebige Einrichtung handeln, welche ein Sterilisationsmittel verdampft und die besagte Sterilisationsmischung aus dem Sterilisationsmittel und dem gasförmigen Medium herstellt. So kann dieser Verdampfer beispielsweise eine Heizoberfläche aufweisen, an der das Sterilisationsmittel verdampft wird, um anschließend mit eintretender Luft gemischt zu werden. Es wäre jedoch auch möglich, dass der Verdampfer derart aufgebaut ist, dass in ihn erhitzte Luft eingeführt wird und in die erhitzte Luft das Sterilisationsmittel eingedüst wird. Auch andere Ausführungsformen von Verdampfern sind jedoch denkbar.

Vorteilhaft weist die Vorrichtung auch eine Steuereinrichtung auf, mittels der die Zuführung der Sterilisationsmischung an die erste Beaufschlagungseinrichtung bzw. in oder an den ersten Bereich und die Zuführung der Sterilisationsmischung an die zweite Beaufschlagungseinrichtung bzw. in oder an einem zweiten Bereich unabhängig voneinander steuerbar sind. Unter einer unabhängigen Steuerung wird daher verstanden, dass wenigstens ein für diese Beaufschlagung charakteristischer Parameter veränderbar ist, wie beispielsweise eine Menge der Sterilisationsmischung, welche auf den Bereich auftrifft, ein Anteil des Sterilisationsmittels innerhalb der Sterilisationsmischung, eine Temperatur der Sterilisationsmischung oder dergleichen.

So könnten beispielsweise die Temperaturen der Sterilisationsmittel oder die Sterilisationsmittelkonzentrationen unabhängig für zwei oder mehrere unterschiedliche beaufschlagte Bereiche gesteuert werden.

Als Sterilisationsmittel wird vorzugsweise Wasserstoffperoxid (H₂O₂) verwendet. Es wäre jedoch auch möglich, andere Substanzen zu verwenden, wie beispielsweise Peressigsäure.

Bei einer weiteren vorteilhaften Ausführungsform weist die Vorrichtung eine weitere Zuführeinrichtung auf, welche der Mischung stromabwärts bezüglich der Verdampfereinheit ein weiteres gasförmiges Medium zuführt. Damit wird hier vorgeschlagen, dass diese weitere Zuführeinrichtung, wie beispielsweise eine weitere Zuführleitung in einer Zuführleitung, welche die Sterilisationsmischung zu der Beaufschlagungseinrichtung führt, das weitere gasförmige Medium, wie beispielsweise Sterilluft, einführen kann. Auf diese Weise ist es möglich, die Konzentration des H₂O₂-Anteils der Sterilisationsmischung zu vermindern. Es wäre jedoch auch möglich, umgekehrt die Sterilisationsmischung in eine weitere Zuführleitung, beispielsweise für Sterilluft, einzuführen. Bei einer weiteren vorteilhaften Ausführungsform ist wenigstens ein Bereich der oben genannten Bereiche aus einer Gruppe von Bereichen ausgewählt, welcher eine Innenoberfläche der Behältnisse, eine Außenoberfläche der Behältnisse, eine Innen- oder Außenoberfläche der Behältnisverschlüsse, einen Mündungsbereich der Behältnisse, einen Bereich eines Behandlungselements, einen Bereich der Vorrichtung, einen Sterilraum oder eine Kombination hieraus enthält.

Weil ein Verdampfer für ein flüssiges Sterilisationsmedium sehr aufwendig und auch kostspielig ist, wird durch die erfindungsgemäße Idee, die Anzahl an Verdampfern auch für komplexere Anlagen reduziert. Im Idealfall ist es möglich, dass nur ein Verdampfer benötigt wird, um eine Vielzahl von unterschiedlichen Bereichen zu steriliseren. Auf diese Weise ist es möglich, im Vergleich zum Stand der Technik wenigstens zwei derartige Verdampfer einzusparen.

Bei einer weiteren vorteilhaften Ausführungsform weist die Vorrichtung eine erste Heizeinrichtung auf, welche das der Verdampfereinheit zuzuführende gasförmige Medium erwärmt. So ist es möglich, dass beispielsweise zugeführte Sterilluft vor ihrer Zuführung in den Verdampfer erwärmt wird. Auf diese Weise kann der Verdampfungsprozess beschleunigt werden.

Vorteilhaft weist die Vorrichtung eine zweite Heizeinrichtung auf, welche die Mischung, d. h. die Sterilisationsmischung erwärmt. Vorteilhaft ist dabei wenigstens eine Heizeinrichtung und bevorzugt beide Heizeinrichtungen steuerbar. Auf diese Weise kann die Temperatur der Sterilisationsmischung ebenfalls beeinflusst werden.

Bei einer weiteren vorteilhaften Ausführungsform ist wenigstens eine Heizeinrichtung ein Wärmetauscher. Dieser Wärmetauscher kann dabei mit Dampf oder einem anderen Medium betrieben werden, um Sterilluft oder auch die Sterilisationsmischung zu erwärmen.

Bei einer weiteren vorteilhaften Ausführungsform weist die Verdampfereinheit eine mit einem fließfähigen Medium erwärmbare Heizeinrichtung zum Verdampfen des Sterilisationsmittels auf. So kann beispielsweise die Heizeinrichtung mit Dampf versorgt werden, um sie auf diese Weise zu erwärmen. Es wären jedoch auch andere Medien zum Erwärmen wie etwa Öl oder Heißluft denkbar

Bei einer weiteren vorteilhaften Ausführungsform ist in zumindest einer Verbindungsleitung zwischen der Verdampfungseinheit und wenigstens einer Beaufschlagungseinrichtung ein regelbares Ventil angeordnet. Durch dieses regelbare Ventil können die Konzentrationsverhältnisse der einzelnen Sterilisationsmischungen angepasst werden.

Weiterhin weist vorteilhaft die Vorrichtung wenigstens eine Sensoreinrichtung auf, welche den Anteil des Sterilisationsmittels in der Sterilisationsmischung bestimmt. Vorteilhaft ist eine Vielzahl derartiger Sensoreinrichtungen vorgesehen, wobei besonders vorteilhaft die Anzahl dieser Sensoreinrichtungen zumindest der Anzahl der zu beaufschlagenden Bereiche entspricht. Daneben kann es sich bei den Sensoreinrichtungen auch um Temperaturmesseinrichtungen handeln.

Bei einer weiteren vorteilhaften Ausführungsform weist die Vorrichtung einen Sterilraum auf, innerhalb dessen die Behältnisse behandelt werden. Bei dieser Anwendung erfolgt vorteilhaft auch die Abfüllung der Behältnisse mit einem Produkt unter sterilen Bedingungen.

Bei einer weiteren vorteilhaften Ausführungsform weist die Vorrichtung eine Transporteinrichtung auf, welche die Behältnisse transportiert. Insbesondere ist dabei eine Transporteinrichtung vorgesehen, welche die Behältnisse wenigstens abschnittsweise durch den besagten Sterilraum transportiert. Vorteilhaft findet ein Transport dabei wenigstens zeitweise während der Sterilisation der Behältnisse statt.

Die vorliegende Erfindung ist weiterhin auf ein Verfahren zum Behandeln von Behältnissen gerichtet, wobei Behältnisse zur Sterilisation mit einer Sterilisationsmischung beaufschlagt werden und diese Sterilisationsmischung mit einer Verdampfereinheit hergestellt wird, und zur Herstellung der Sterilisationsmischung in der Verdampfereinheit, wobei der Verdampfereinheit ein Sterilisationsmittel und ein gasförmiges Medium zugeführt werden, und die erzeugte Sterilisationsmischung aus der Verdampfereinheit abgeführt wird. Erfindungsgemäß wird die aus der Verdampfereinheit abgeführte Sterilisationsmischung zu wenigstens zwei unterschiedlichen zu sterilisierenden Bereichen zugeführt. Einer dieser Bereiche kann dabei ein Bereich des Behältnisses oder eines Bestandteils des Behältnisses sein und ein zweiter Bereich, beispielsweise ein Behandlungselement oder der oben erwähnte Isolator selbst.

Durch die erfindungsgemäßen Vorgehensweisen ist es möglich, komplizierte Bauteile, wie beispielsweise den Verdampfer und die damit verbundenen Überwachungseinrichtungen zu reduzieren. Auf diese Weise kann insgesamt eine Reduzierung der Kosten und auch eine Vereinfachung des verfahrenstechnischen Aufbaus erreicht werden. Daneben wird bei vielen Anlagen eine sehr geringe Menge an flüssigen Sterilisationsmedien benötigt. Diese sehr geringen Mengen sind nur schwer mit hinreichender Genauigkeit mit einem einzelnen Verdampfer zu dosieren. Durch die vorteilhaften Ausgestaltungen ist es möglich, dass die gesamte Menge an Sterilisationsmedien in einem Verdampfer verdampft wird und diese Menge immer in einem Bereich liegt, der relativ einfach zu dosieren ist. Durch die Reduzierung der Anzahl an Verdampfern, ist es auch möglich, die Kosten für Wartungs- und Inspektionsaufgaben zu reduzieren. Daneben kann durch die Reduzierung der Anzahl der Verdampfereinheiten auch Platz in den Ventilknoten eingespart werden, wodurch die Anlagen insgesamt weniger Aufstellungsflächen benötigen.

Die Verdampfeinheiten können sowohl elektrisch, als auch mit Dampf erhitzt werden. Oftmals werden kleine Verdampfereinheiten elektrisch erhitzt, da die Realisierung eines Dampfanschlusses oftmals nicht rentabel ist. Elektrisch betriebene Verdampfereinheiten weisen jedoch wesentliche Nachteile gegenüber dampfbetriebenen auf. Durch die Reduzierung der genannten kleinen Verdampfereinheiten auf einen "großen" Verdampfer wird die Erhitzung mittels Dampf ökonomisch sinnvoll. Auf diese Weise können wiederum Einsparungen im Bau der Anlage erreicht werden.

Weitere vorteilhafte Ausführungsformen ergeben sich aus der beigefügten Figur.

Hierbei zeigt:

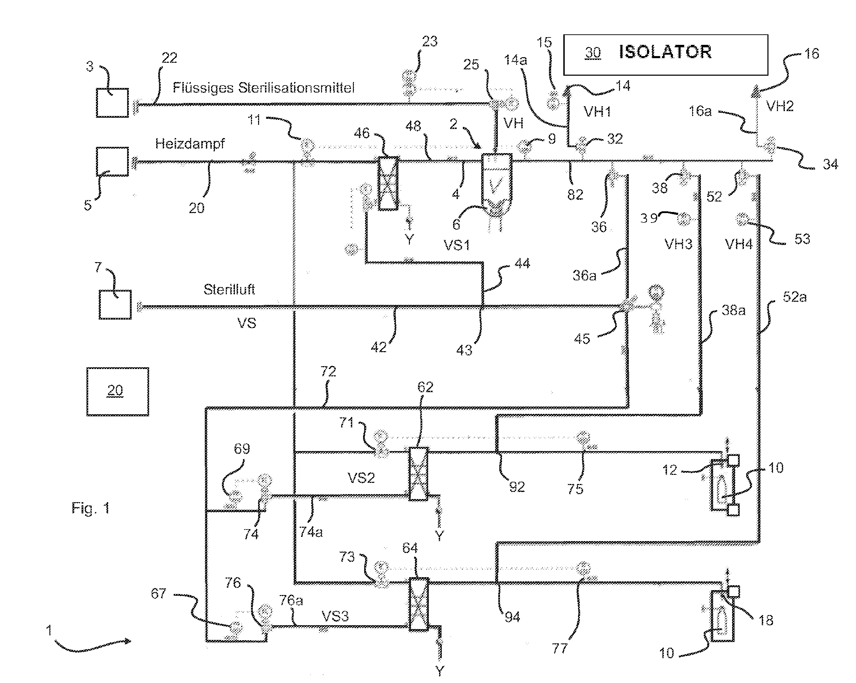

- Fig. 1: eine Skizze zur Veranschaulichung einer erfindungsgemäßen Vorrichtung.

Fig. 1 zeigt eine Skizze zur Veranschaulichung einer erfindungsgemäßen Vorrichtung 1. In der Skizze zeigen die jeweils durchgezogenen Linien solche Leitungen, entlang derer ein fließfähiges Medium gefördert wird, wie beispielsweise Sterilluft, Heizdampf oder das flüssige Sterilisationsmittel. Dabei wird über ein Reservoir 3 das flüssige Sterilisationsmittel zur Verfügung gestellt, über ein Reservoir 5 Heizdampf, und über ein Reservoir 7 Sterilluft (Volumenstrom VS). Da, wie oben erwähnt, ein Verdampfer für ein flüssiges Sterilisationsmedium sehr aufwändig und auch kostspielig ist, wird durch diese Idee die Zahl der Verdampfer reduziert, und im Idealfall wird nur eine Verdampfereinheit 2 benötigt. Auf diese Weise können mindestens zwei Verdampfer eingespart werden. Das Bezugszeichen 6 kennzeichnet eine Heizeinrichtung der Verdampfereinheit 2.

Ausgehend von dem Reservoir 7 wird (insbesondere vor einem Arbeitsbetrieb der Vorrichtung) ein Sterilluftstrom VS über eine Leitung 42 und einen Verteilpunkt 43 in eine Leitung 44 gefördert (VS1) und über eine Heizeinrichtung 46 auf eine definierte Temperatur gebracht. Diese Temperatur liegt dabei zwischen 100°C und 145°C und bevorzugt zwischen 110°C und 130°C. Der Sterilluftstrom strömt anschließend über einen Leitungsteil 48 einer in ihrer Gesamtheit mit 4 bezeichneten Leitung und gelangt zu der zentralen Verdampfereinheit 2. Anschließend wird der Sterilluftstrom VS auf alle angeschlossenen Rohrleitungen, wie beispielsweise die Leitungen 82, 52a, 36a, 14a und 16a verteilt. Auf diese Weise wird das Rohrleitungssystem erhitzt und getrocknet. Das Bezugszeichen 15 kennzeichnet eine in der Leitung 14a befindliche Sensoreinrichtung wie etwa einen Temperatursensor, einen Durchflusssensor oder einen Sensor, der eine Konzentration des Sterilisationsmittels bestimmt.

Sobald alle genannten Rohrleitungen eine definierte Temperatur erreicht haben und für eine bestimmte Zeit getrocknet wurden, wird ein flüssiges Sterilisationsmedium, ausgehend von dem Reservoir 3 und die Leitung 22 in die Verdampfereinheit 2 geleitet. Das dabei verdampfte Sterilisationsmedium wird auf alle Rohrleitungen verteilt und sterilisiert diese dadurch (ausgehend von der Leitung 82). Durch diese Vorgehensweise kann die komplette Vorrichtung sterilisiert werden, also insbesondere auch der Isolator 30 und alle gezeigten Rohrleitungen. Das Bezugszeichen 25 kennzeichnet ein regelbares Ventil, welches von einer Sensoreinrichtung 23 gesteuert wird bzw. in Reaktion auf Signale dieser Sensoreinrichtung gesteuert wird.

Nach dieser Sterilisation kann die Abfüllung beginnen. Um nun die Flaschen 10 und ggfs. auch deren Verschlüsse zu sterilisieren, wird in dem Verdampfer 2 ein hoch angereichertes Gas erzeugt. Dieses Gas wird anschließend über Regelventile 45, 36, 38, 52 und über Durchflussmesser 53, 39 der Sterilluft, die für die Behältnisbehandlung benötigt wird zugemischt. Auf diese Weise stellt sich ein Gasluftgemisch mit der geforderten Gaskonzentration zur Entkeimung ein. Die Bezugszeichen 36a, 38a, 52a beziehen sich auf Verbindungsleitungen, die sich über Knotenpunkte an die Leitung 82 anschließen.

Um beispielsweise die Behältnisse zu sterilisieren, wird der Gasvolumenstrom aus dem Verdampfer, der beispielsweise eine H₂O₂-Konzentration von 6000 ppm aufweisen kann (VH3) mit dem Sterilluftvolumenstrom VS2 gemischt. Dieser Sterilluftstrom VS2 kann dabei durch den Erwärmer 62 wiederum mit Heizdampf erwärmt werden. Der Gasvolumenstrom VH3 kann dabei jedoch auch 0 kg/h betragen. Auf diese Weise stellt sich das entsprechende Mischgas ein, welches für die Behältnisbehandlung erforderlich ist (etwa 4000 ppm). Die geforderte Temperatur zur Behältnisbehandlung wird, wie oben erwähnt, mittels der Temperatur von VS2 über die Heizeinrichtung 62 eingestellt. Über eine Sensoreinrichtung 75 und eine Ventileinrichtung 71 kann dabei die Temperatur gesteuert werden. Hier handelt es sich bei der Sensoreinrichtung 75 um eine Temperaturmesseinrichtung. Die Ventileinrichtung 71 steuert die Zufuhr von Heizdampf in die Heizeinrichtung 62. Über eine Sensoreinrichtung 69 und eine Ventileinrichtung 74 kann die Zufuhr der Sterilisationsmischung in die Heizeinrichtung 62 und damit auch zu der Beaufschlagungseinrichtung 12, welche das Behältnis 10 beaufschlägt, gesteuert werden.

Das Bezugszeichen 92 kennzeichnet einen Knotenpunkt, an dem auch stromabwärts der Heizeinrichtung 62 eine Sterilisationsmischung zugeführt werden kann. Die Bezugszeichen VH1, VH2, VH3, VH4 bezeichnen die Sterilisationsmischungsströme, die von der Leitung 82 abzweigen und zu den Beaufschlagungseinrichtungen 12, 14, 16 und 18 führen.

In entsprechender Weise läuft auch die Mischung im Falle der Beaufschlagungseinrichtung 18 ab. Hier ist ebenfalls eine Heizeinrichtung 64 vorgesehen, welche mit Heizdampf betrieben wird, sowie eine Ventileinrichtung 73, die wiederum die Zufuhr an Heizdampf steuert, wobei hierzu auch die Sensoreinrichtung 77 die Temperatur misst. Auch hier wird der Volumenstrom VS3 über eine Ventileinrichtung 76 und einen Sensor 67 gesteuert. Das Bezugszeichen 72 kennzeichnet eine Zufuhrleitung, welche sich auf die beiden Leitungen 74a und 76a aufteilt, welche die Sterilluftvolumenstrom VS2 und VS3 jeweils an die Heizeinrichtung 62 und 64 leiten. Am Knotenpunkt 94 kann entsprechend Knotenpunkt 92 stromabwärts der Heizeinrichtung 64 eine Sterilisationsmischung zugeführt werden.

Die Temperatur des Volumenstroms VS1 kann in einem großen Bandbereich gewählt werden, liegt aber vorteilhaft über dem Taupunkt des Sterilisationsmediums (zwischen 70° C und 150° C).

Über die Ventileinrichtungen 32 und 34 und die Leitungen 14a und 16a kann der Isolator 30 über die Beaufschlagungseinrichtungen 14 und 16 mit dem Sterilisationsmedium beaufschlagt werden. Man erkennt hier, dass hierzu die Sterilisationsmischung unmittelbar nach dem Verdampfer, d. h. in der höchsten Konzentration verwendet wird. Das Bezugszeichen 9 kennzeichnet eine weitere Temperaturmesseinrichtung, welche ein Ventil 11 steuert, um auf diese Weise auch die Temperatur der in die Verdampfereinheit 2 eingeführten Sterilisationsmischung einstellen zu können. Das Bezugszeichen 15 bezeichnet eine Sensoreinrichtung zur Konzentrations- und Temperaturmessung, die bevorzugt direkt vor der Beaufschlagungseinrichtung 14 angeordnet ist. Ebenso ist hier eine Volumenstrommessung möglich. Entsprechende Sensoren (nicht gezeigt) können sich auch in Leitung 16a, bevorzugt direkt vor der Beaufschlagungseinrichtung, angeordnet sein.

Mit der dargestellten Vorgehensweise können zeitlich parallel beliebig viele unterschiedliche Gaskonzentrationen bei unterschiedlichen Temperaturen erzeugt werden, und zur Sterilisation von unterschiedlichen Packstoffen, Behältnissen, Verschlüssen bzw. Elementen der Vorrichtung während der Produktion und anderen Betriebszuständen verwendet werden.

Damit weist die erfindungsgemäße Vorrichtung auch eine Regelungseinrichtung in Form einer Vielzahl von Regelventilen und Sensoreinrichtungen auf, mit denen jeweils charakteristische Parameter, wie die Temperatur, Konzentrations- und Sterilisationsmedien und der gleichen separat für die einzelnen Beaufschlagungseinrichtungen 12, 18, 14 und 16 geregelt werden können. Das Bezugszeichen 20 kennzeichnet eine (zentrale) Steuereinrichtung, mittels derer die einzelnen Ventile zentral und gegebenenfalls auch nach Vorgaben eines Maschinenbedieners angesteuert werden können.

Die Anmelderin behält sich vor, einzelne oder sämtliche in den Anmeldungsunterlagen offenbarte Merkmale als erfindungswesentlich zu beanspruchen, sofern sie einzeln oder in Kombination gegenüber dem Stand der Technik neu sind.

### Bezugszeichenliste

- 1: Vorrichtung
- 2: Verdampfereinheit
- 3: Reservoir für Sterilisationsmittel
- 4: Zuführleitung
- 5: Reservoir für Heizdampf
- 6: Heizeinrichtung der Verdampfereinheit 2
- 7: Reservoir für Sterilluft
- 9: Temperaturmesseinrichtung
- 10: Flaschen, Behältnis
- 11: Ventil
- 12, 14, 16, 18: Beaufschlagungseinrichtung
- 14a: Leitung
- 15, 23: Sensoreinrichtung
- 16a: Leitung
- 22: Zuführungsleitung
- 30: Isolator
- 25, 32, 34: Ventileinrichtung
- 42,44: Leitung
- 43: Verteilpunkt
- 45, 36, 38, 52: Regelventile
- 48: Leitungsteil
- 53, 39: Durchflussmesser
- 46, 62, 64: Heizeinrichtung
- 67,69: Sensoreinrichtung
- 72: Zuführleitung
- 71, 73, 74, 75, 76, 77: Ventileinrichtung
- 74a, 76a: Leitung
- 82, 52a, 36a,: Leitungen
- 38a,14a, 16a: Leitungen
- 92, 94: Knotenpunkt
- VH3: Sterilluftvolumenstrom
- VH: Sterilisationsmedium
- VS, VS1 - VS3: Sterilluftvolumenstrom

## Patentansprüche

1. Vorrichtung (1) zum Sterilisieren von Verpackungsmitteln und insbesondere Behältnissen mit einer Verdampfereinheit (2), welche eine Zuführungseinrichtung (22) für ein Sterilisationsmittel und eine Zuführleitung (4) für ein gasförmiges Medium aufweist, wobei die Verdampfereinheit das Sterilisationsmittel in das gasförmige Medium einbringt und weiterhin eine Abführeinrichtung (82) zum Abführen einer Sterilisationsmischung aus dem Sterilisationsmittel und dem gasförmigen Medium aufweist und mit einer ersten Beaufschlagungseinrichtung (12), welche wenigstens einen ersten Bereich mit der Sterilisationsmischung beaufschlagt, wobei die erste Beaufschlagungseinrichtung (12) mit der Verdampfereinheit (2) in Strömungsverbindung steht,
**dadurch gekennzeichnet, dass**
die Vorrichtung (1) eine zweite Beaufschlagungseinrichtung (14) aufweist, welche wenigstens einen zweiten sich von dem ersten Bereich unterscheidenden Bereich mit der Sterilisationsmischung beaufschlagt, wobei auch die zweite Beaufschlagungseinrichtung (14) mit der Verdampfereinheit (2) in Strömungsverbindung steht.

2. Vorrichtung (1) nach Anspruch 1,
**dadurch gekennzeichnet, dass**
die Vorrichtung auch eine Steuereinrichtung aufweist, mittels der die Zuführung der Sterilisationsmischung an die erste Beaufschlagungseinrichtung (12) und die Zuführung der Sterilisationsmischung in oder an die zweite Beaufschlagungseinrichtung (14) unabhängig voneinander steuerbar sind.

3. Vorrichtung (1) nach Anspruch 1,
**dadurch gekennzeichnet, dass**
die Vorrichtung eine weitere Zuführeinrichtung (42) aufweist, welche der Sterilisationsmischung stromabwärts bezüglich der Verdampfereinheit (2) ein gasförmiges Medium zuführt.

4. Vorrichtung nach wenigstens einem der vorangegangenen Ansprüche,
**dadurch gekennzeichnet, dass**
wenigstens ein Bereich aus einer Gruppe von Bereichen ausgewählt ist, welche eine Innenoberfläche der Behältnisse, eine Außenoberfläche der Behältnisse, die Oberfläche von Behältnisverschlüssen, einen Bereich eines Behandlungselements, einen Bereich der Vorrichtung, einen Sterilraum, Kombinationen hieraus oder dergleichen enthält.

5. Vorrichtung nach wenigstens einem der vorangegangenen Ansprüche,
**dadurch gekennzeichnet, dass**
die Vorrichtung eine erste Heizeinrichtung (46) aufweist, welche das der Verdampfereinheit (2) zuzuführende gasförmige Medium erwärmt.

6. Vorrichtung nach wenigstens einem der vorangegangenen Ansprüche,
**dadurch gekennzeichnet, dass**
die Vorrichtung (1) eine zweite Heizeinrichtung (62, 64) aufweist, welche die Mischung erwärmt.

7. Vorrichtung nach Anspruch 5,
**dadurch gekennzeichnet, dass**
wenigstens eine Heizeinrichtung (46, 62, 64) ein Wärmetauscher (46, 62, 64) ist.

8. Vorrichtung nach wenigstens einem der vorangegangenen Ansprüche,
**dadurch gekennzeichnet, dass**
die Verdampfereinheit (2) eine mit einem fließfähigen Medium erwärmbare Heizeinrichtung (6) zum Verdampfen des Sterilisationsmittels aufweist.

9. Vorrichtung nach wenigstens einem der vorangegangenen Ansprüche,
**dadurch gekennzeichnet, dass**
in wenigstens einer Verbindungsleitung (36a, 38a, 52a) zwischen der Verdampfereinheit (2) und einer Beaufschlagungseinrichtung ein regelbares Ventil angeordnet ist.

10. Vorrichtung nach wenigstens einem der vorangegangenen Ansprüche,
**dadurch gekennzeichnet, dass**
die Vorrichtung (1) einen Sterilraum (30) aufweist, innerhalb dessen die Behältnisse (10) behandelt werden.

11. Vorrichtung nach wenigstens einem der vorangegangenen Ansprüche,
**dadurch gekennzeichnet, dass**
die Vorrichtung eine Transporteinrichtung aufweist, welche die Behältnisse (10) transportiert.

12. Verfahren zum Behandeln von Verpackungsmitteln und insbesondere von Behältnissen (10), wobei Behältnisse (10) zur Sterilisation mit einer Sterilisationsmischung beaufschlagt werden und diese Sterilisationsmischung in einer Verdampfereinheit (2) hergestellt wird, und zur Herstellung der Sterilisationsmischung in der Verdampfereinheit (2) dieser Verdampfereinheit (2) ein Sterilisationsmittel und ein gasförmiges Medium zugeführt werden und die erzeugte Sterilisationsmischung aus der Verdampfereinheit (2) abgeführt wird,
**dadurch gekennzeichnet, dass**
die aus der Verdampfereinheit (2) abgeführte Sterilisationsmischung wenigstens zwei unterschiedlichen zu sterilisierenden Bereichen zugeführt wird.
